# EUROPÄISCHE PATENTSCHRIFT

(11) **EP 1 914 547 B1**
(45) Veröffentlichungstag und Bekanntmachung des Hinweises auf die Patenterteilung: **06.03.2013**
(21) Anmeldenummer: 07023722.7
(22) Anmeldetag: 12.10.2005
(51) Int. Cl.: G01N 33/487

(54) **Vorrichtung, Messgerät und Verfahren zur Handhabung mikrofluidischer Plattformen**
Device, measuring apparatus and method for drawing and examining or manipulating sample liquid on a microfluidic platform
Dispositif, appareil de mesure et procédé pour la réception et l'analyse ou la manipulation d'un liquide de prélèvement sur une plateforme microfluidique

(30) Priorität: 13.10.2004 DE 102004050062
(43) Veröffentlichungstag der Anmeldung: 23.04.2008
(62) Teilanmeldung aus: 05022253.8
(73) Patentinhaber: Boehringer Ingelheim microParts GmbH, 44227 Dortmund (DE)
(72) Erfinder: Kadel, Klaus, Dr., 58453 Witten (DE); Blankenstein, Gert, Dr., Arlington, MA 02476 (US); Dechant, Christian, 88339 Bad Waldsee (DE)

(56) Entgegenhaltungen:
- DE-A1- 4 326 339
- DE-A1- 10 244 775
- DE-A1- 19 815 684
- US-A- 5 514 152
- US-A1- 2003 024 811

## Beschreibung

Die vorliegende Erfindung betrifft eine Vorrichtung mit einer Vielzahl von mikrofluidischen Plattformen zur Aufnahme und insbesondere Untersuchung oder Manipulation von Probenflüssigkeit gemäß dem Oberbegriff des Anspruchs 1, ein Messgerät zur Untersuchung von Probenflüssigkeit, beispielsweise Blut zur Glucose-Bestimmung, gemäß dem Oberbegriff des Anspruchs 11 sowie ein Verfahren zur Untersuchung oder Manipulation von Probenflüssigkeit gemäß dem Oberbegriff des Anspruchs 13.

Der Begriff "mikrofluidische Plattform" - nachfolgend oft kurz als Plattform bezeichnet - umfasst bei der vorliegenden Erfindung Probenträger, Teststreifen oder dgl., die insbesondere durch Kapillarkräfte eine zu untersuchende oder zu manipulierende Probenflüssigkeit aufnehmen können. Insbesondere dienen die Plattformen zur Durchführung einzelner Tests oder Messungen, beispielsweise der Glucose-Bestimmung von Blut. Jedoch können die Plattformen auch jeder sonstigen Untersuchung, insbesondere von Körperflüssigkeiten von Menschen oder Tieren als Probenflüssigkeit dienen. Zur Untersuchung bzw. Manipulation von Probenflüssigkeit weisen die Plattformen insbesondere Reagenzien, Filter oder dgl. für die Probenflüssigkeit auf. Zur Bevorratung bzw. Lagerung sind die Plattformen üblicherweise verschlossen.

Derartige Plattformen werden beispielsweise in Form von Teststreifen zur Bestimmung des Blutzuckerspiegels (Glucose-Bestimmung) verwendet. Bisher ist es hierbei erforderlich, einen einzelnen Teststreifen aus einer Vorratspackung zu entnehmen und mit Blut als Probenflüssigkeit zu benetzen, die dann von dem Teststreifen aufgenommen wird. Anschließend muss der Teststreifen manuell in ein entsprechendes Mess- bzw. Testgerät eingesetzt werden, das dann zunächst anzeigt, ob in ausreichender Menge Blut aufgenommen wurde und schließlich den Glucosewert bestimmt. Anschließend muss der Teststreifen wieder entnommen und entsorgt werden. Der beschriebene Ablauf ist aufwendig und mit gewissen Unsicherheiten verbunden. Die Handhabung des verhältnismäßig kleinen Teststreifens ist insbesondere für ungeübte Benutzer schwierig. Es besteht das Risiko einer unerwünschten Kontaminierung. Des weiteren wird oftmals Blut in nicht ausreichender Menge aufgenommen, was jedoch erst nach dem Einsetzen des Teststreifens in das Messgerät feststellbar ist, so dass dann die gleiche Prozedur noch einmal durchlaufen werden muss.

Aus der WO 03/045557 A2, die den Ausgangspunkt der vorliegenden Erfindung bildet, ist eine bandartige Anordnung von einer Vielzahl von Plattformen bekannt, die hintereinander für eine Vielzahl von unmittelbar aufeinander folgenden, automatisierten Untersuchungen bzw. Manipulation von Probenflüssigkeiten einsetzbar sind. Die bekannte Anordnung ist jedoch nicht zur einzelweisen Untersuchung bzw. Manipulation von Probenflüssigkeit vorgesehen oder geeignet.

Die US 5,514,152 A betrifft eine Vorrichtung mit einer Vielzahl mikrofluidischer Plattformen zur Aufnahme und Untersuchung von Probeflüssigkeit. Die Plattformen sind im verschlossenen Zustand miteinander verbunden und können jeweils einzelweise abgetrennt und geöffnet werden. Weiterhin wird ein Gerät und ein Verfahren zur Verwendung der Vorrichtung offenbart.

Der vorliegenden Erfindung liegt die Aufgabe zugrunde, eine Vorrichtung mit einer Vielzahl von mikrofluidischen Plattformen zur Aufnahme und insbesondere Untersuchung oder Manipulation von Probenflüssigkeiten, ein Messgerät zur Untersuchung von Probenflüssigkeit mittels einer mikrofluidischen Plattform und ein Verfahren zur Untersuchung oder Manipulation von Probenflüssigkeit mittels einer mikrofluidischen Plattform anzugeben, wobei eine bedarfsgerechte einzelweise Aufnahme und insbesondere Untersuchung oder Manipulation von Probenflüssigkeit, vorzugsweise also einzelne Untersuchungen bzw. Manipulationen unabhängig von vorherigen Untersuchungen bzw. Manipulationen, ermöglicht werden, wobei insbesondere aus hygienischen Gründen jede benutzte Plattform unmittelbar nach ihrer Benutzung und unabhängig von den anderen Plattformen entsorgt werden kann.

Die obige Aufgabe wird durch eine Vorrichtung gemäß Anspruch 1, ein Messgerät gemäß Anspruch 11 oder ein Verfahren gemäß Anspruch 13 gelöst. Vorteilhafte Weiterbildungen sind Gegenstand der Unteransprüche.

Ein Aspekt der vorliegenden Erfindung liegt darin, bei einer Vielzahl von Plattformen, die insbesondere in einer bandförmigen, beispielsweise aufgewickelten oder gestapelten Anordnung vorliegen, die Plattformen einzelweise zu separieren und einzelweise zu öffnen, so dass bedarfsgerecht die Plattformen einzelweise zur Aufnahme und insbesondere Untersuchung oder Manipulation von Probenflüssigkeit eingesetzt und anschließend - unabhängig von den noch nicht benutzten Plattformen - entsorgt werden können. Dies ermöglicht eine wesentlich vereinfachte Handhabung für einen Benutzer, der beispielsweise in gewissen Zeitabständen seinen Blutzuckerspiegel überprüfen muss. Erfindungsgemäß ist es dann nämlich möglich, ein die Vielzahl von Plattformen enthaltendes Messgerät einzusetzen, das die Bestimmung des Blutzuckerwerts (Glucose-Bestimmung) bedarfsgerecht gestattet, ohne dass der Benutzer zunächst einen einzelnen Teststreifen nach der Aufnahme von Blut in das Messgerät einführen und anschließend wieder entfernen muss. Vielmehr ist das Messgerät nach einem vorzugsweise automatisierten Öffnen einer Plattform direkt zur Aufnahme von Blut und beispielsweise Bestimmung des Glucosewerts einsetzbar, wobei unmittelbar nach der Bestimmung die benutzte Plattform vorzugsweise direkt ausgegeben und entsorgt werden kann. Dies ermöglicht eine optimale bedarfsgerechte und hygienische Benutzung bei einfacher Handhabung.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. Es zeigt:
- Fig. 1 :: eine ausschnittsweise Draufsicht einer vorschlagsgemäßen Vorrichtung
- Fig. 2 :: einen Schnitt entlang Linie VI-VI von Fig. 1;
- Fig. 3 :: eine Darstellung der Vorrichtung beim Öffnen einer Plattform;
- Fig. 4 :: eine schematische Darstellung eines Messgeräts mit der Vorrichtung während der Aufnahme von Probenflüssigkeit;
- Fig. 5 :: das Messgerät beim Auswerfen einer benutzten Plattform;
- Fig. 6 :: eine schematische Darstellung eines Messgeräts mit einer vorschlagsgemäßen Vorrichtung gemäß einer zweiten Ausführungsform;
- Fig. 7 :: die zweite Ausführungsform mit einer geöffneten Plattform zur Aufnahme von Probenflüssigkeit; und
- Fig. 8 :: die zweite Ausführungsform nach der Untersuchung von Probenflüssigkeit beim Auswerfen einer benutzten Plattform.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

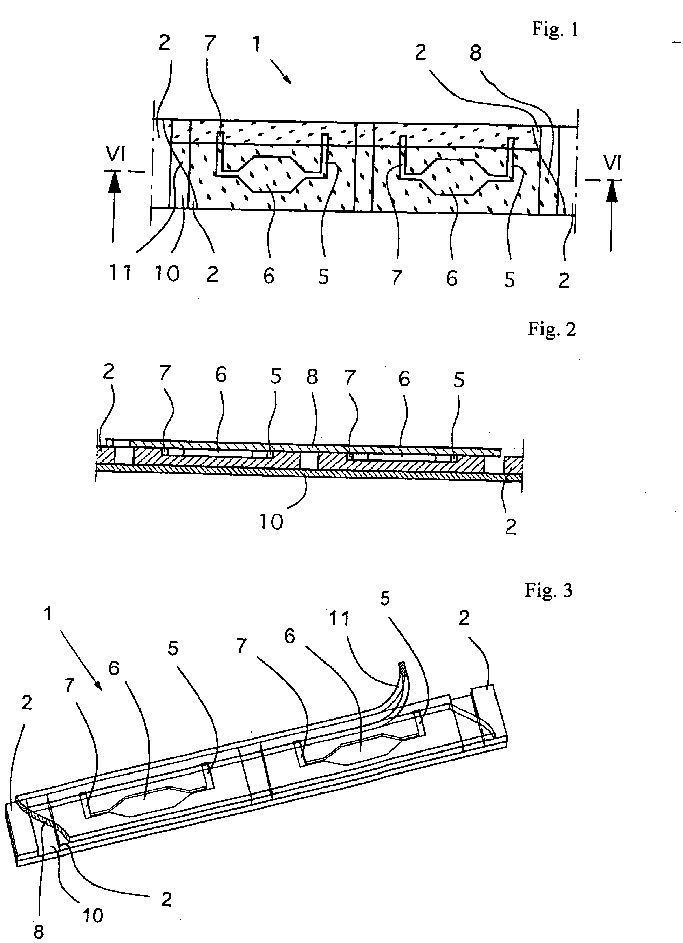

Fig. 1 zeigt eine vorschlagsgemäße Vorrichtung 1 mit einer Vielzahl von mikrofluidischen Plattformen 2 im eingangs genannten Sinne. Die Plattformen 2 dienen der Aufnahme und Untersuchung oder Manipulation von Probenflüssigkeit 3, beispielsweise Blut zur Glucose-Bestimmung.

Jede Plattform 2 weist einen Aufnahmekanal 5 zur Aufnahme von Probenflüssigkeit 3, eine sich anschließende Probenkammer 6, die einer Untersuchung und/oder Manipulation der Probenflüssigkeit 3 - bedarfsweise unter Einwirkung von einem vorher in die Probenkammer 6 eingebrachten, nicht dargestellten, insbesondere eingetrockneten Reagenz oder dgl. - dient, und einen sich an die Probenkammer 6 anschließenden Entlüftungskanal 7 auf.

Die Plattformen 2 können je nach Bedarf und Ausbildung beispielsweise durch Schneiden, Brechen oder Reißen im Bereich ihrer Verbindungen 4 - wahlweise manuell oder insbesondere mittels einer geeigneten Einrichtung - bedarfsgerecht und einfach voneinander getrennt werden, wobei die vorzugsweise vorgesehenen Sollbruchstellen jedoch nicht unbedingt erforderlich sind.

Fig. 1 und Fig. 2 zeigen eine Ausführungsform der vorschlagsgemäßen Vorrichtung 1 mit einer Vielzahl von Plattformen 2; Fig. 1 in einer ausschnittsweisen Draufsicht und Fig. 2 in einer ausschnittsweisen Seitenansicht.

Bei dieser Ausführungsform sind die wahlweise steif oder ggf. flexibel ausgebildeten Plattformen 2 nicht unmittelbar miteinander verbunden, sondern auf einem gemeinsamen Träger 10 angeordnet, der vorzugsweise bandartig ausgebildet ist und insbesondere gleichzeitig als Transport- bzw. Förderband dient. Auch bei dieser Ausführungsform sind die Plattformen 2 vorzugsweise zumindest im wesentlichen bandförmig hintereinander angeordnet.

Dabei verlaufen der Aufnahmekanal 5 und der Entlüftungskanal 7, ausschließlich in der jeweiligen Plattform 2 und erstrecken sich nicht in die benachbarte Plattform 2. Statt dessen enden die beiden Kanäle 5, 7 im Bereich eines Längsrands innerhalb der jeweiligen Plattform 2. Bei der Ausführungsform ist die Probenkammer 6 im wesentlichen in der Mitte der jeweiligen Plattform 2 angeordnet.

Die Plattformen 2 sind wiederum durch eine Abdeckung 8 überdeckt, die beim Darstellungsbeispiel durchgehend ausgebildet ist, bedarfsweise jedoch auch aus separaten Stücken, die den einzelnen Plattformen 2 zugeordnet sind, gebildet sein kann.

Die Abdeckung 8 besteht wieder vorzugsweise aus Folie oder aus einem sonstigen geeigneten Material. Dabei deckt die Abdeckung 8 alle Kavitäten, wie den Aufnahmekanal 5, die Probenkammer 6 und den Entlüftungskanal 7, jeder einzelnen Plattform 2 im geschlossenen Zustand ab. Beim Darstellungsbeispiel umfasst die Abdeckung 8 einen separat lösbaren oder öffenbaren Bereich, der vorzugsweise als durchgehender Längsstreifen 11 ausgebildet ist, um ein einzelweises Öffnen der Plattformen 2 zur Aufnahme von Probenflüssigkeit 3 zu ermöglichen.

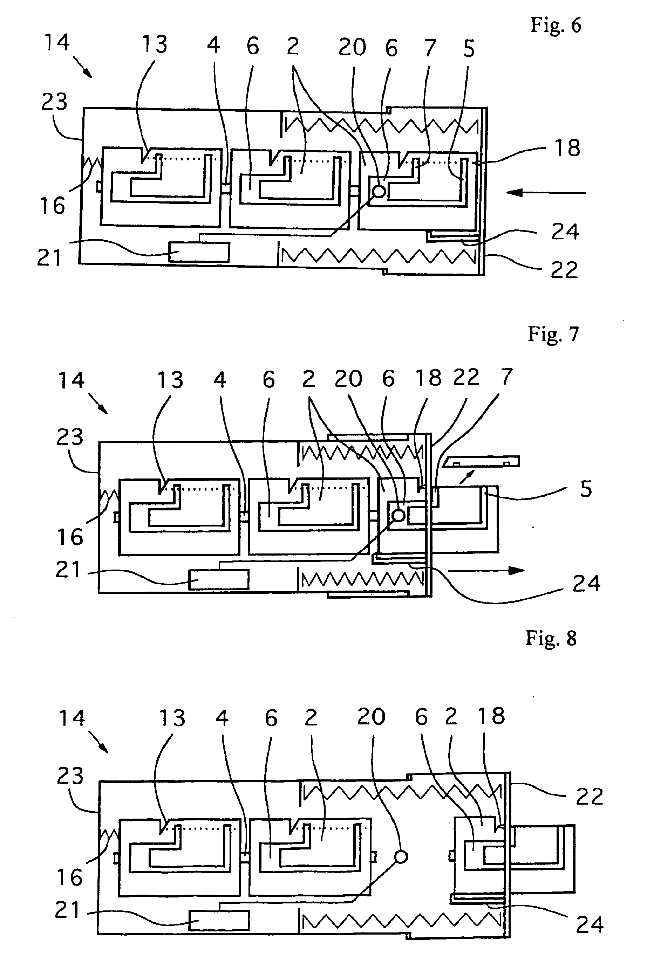

Bei der Ausführungsform ist die Abdeckung 8 hinsichtlich des Streifens 11, derart ausgebildet, dass sie von den Plattformen 2 wieder lösbar ist, um Aufnahmekanal 5 und Entlüftungskanal 7 einzelweise öffnen zu können, ohne jedoch den Aufnahmekanal 5 und den Entlüftungskanal 7 vollständig aufzudecken und ohne die Probenkammer 6 aufzudecken, wie in Fig. 7 schematisch dargestellt. So bleibt also die gewünschte Funktionalität der einzelnen Plattformen 2 zur Aufnahme und insbesondere Untersuchung oder Manipulation von Probenflüssigkeit 3 erhalten.

Um ein einfaches Trennen der Plattform 2 vom Träger 10 zu ermöglichen, sind die Plattformen 2 einzelweise vom Träger 10 abhebbar oder insbesondere abziehbar. Vorzugsweise besteht eine entsprechend lösbare Klebeverbindung zwischen den Plattformen 2 und dem Träger 10.

Alternativ kann der Träger 10 auch aus dem gleichen Material wie die Plattformen 2 und ggf. sogar einstückig mit diesen hergestellt sein.

Die Vorrichtung 1 gemäß der Ausführungsform ist vorzugsweise derart ausgebildet, dass sie bzw. die noch nicht separierten Plattformen 2 aufwickelbar oder stapelbar sind, insbesondere aufgrund eigener Flexibilität der Plattformen 2 und/oder des Trägers 10 und/oder aufgrund entsprechend flexibler Verbindungen 4.

Die Vorrichtung 1 bzw. die Plattformen 2 ist bzw. sind vorzugsweise mit mechanischen und/oder optischen Markierungen, Aussparungen, Löchern, Schwächungen, oder Hinterschneidungen 13 (Fig. 6) versehen, um das Fördern, Positionieren, Erfassen und/oder Separieren einzelner Plattformen 2 zu ermöglichen oder zu erleichtern.

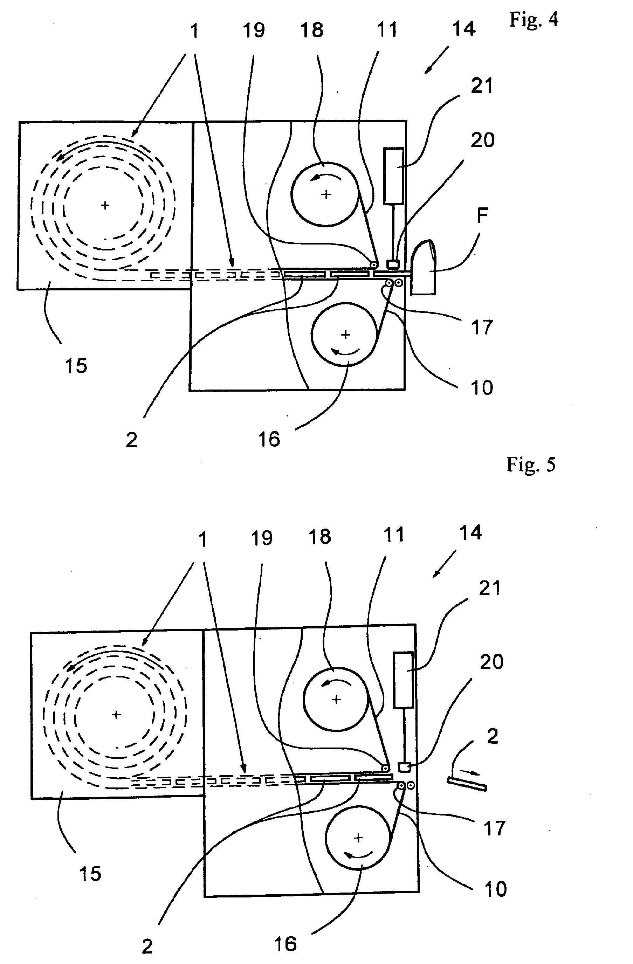

Nachfolgend wird zunächst eine erste Ausführungsform eines Messgeräts 14 für die Vorrichtung 1 gemäß der beschriebenen Ausführungsform anhand von Fig. 4 und 5 näher erläutert, bevor auf eine weitere Ausführungsform der Vorrichtung 1 näher eingegangen wird.

Das Messgerät 14 dient der Aufnahme einer Vorrichtung 1 mit mehreren Plattformen 2. Das Messgerät 14 weist ein wechselbares Magazin 15 zur Aufnahme und Bevorratung der Vorrichtung 1 bzw. Plattformen 2 auf.

Das Messgerät 14 weist eine Förder- bzw. Vorschubeinrichtung 16 zur Förderung der Vorrichtung 1 gemäß der genannten Ausführungsform auf. Der Träger 10 wird insbesondere in der Art eines Förderbands eingesetzt, wobei ein nicht dargestelltes Zackenrad oder Zahnrad zum definierten Vortrieb und Positionieren des Trägers 10 und damit der Plattformen 2 verwendbar ist. Der Antrieb kann mechanisch oder elektrisch, insbesondere mittels eines Schrittmotors oder dgl., erfolgen. Beim Darstellungsbeispiel wickelt die Vorschubeinrichtung 16 den ggf. um eine Führungsrolle 17 geführten Träger 10 bedarfsgerecht auf.

Das Messgerät 14 weist ferner eine Öffnungseinrichtung 18 zum einzelweisen Öffnen der Plattform 2 zur Aufnahme von Probenflüssigkeit 3 auf. Beim Darstellungsbeispiel erfolgt dies durch Abziehen des Streifens 11 von der zu öffnenden Plattform 2. Vorzugsweise wird der Streifen 11 der Abdeckung 8 über eine Führungsrolle 19 geführt und direkt von der Öffnungseinrichtung 18 aufgewickelt.

Beim Darstellungsbeispiel ist die geöffnete Plattform 2 bereits in eine vorgeschobene Stellung gefördert, in der die Plattform 2 zumindest mit einem den Aufnahmekanal 5 enthaltenden Bereich aus dem Messgerät 14 vorragt oder freiliegt, so dass dann die Aufnahme der Probenflüssigkeit 3 erfolgen kann, wie in Fig. 4 beispielhaft durch den Finger F angedeutet, insbesondere also keine direkte manuelle Handhabung der Plattform 2 zur Aufnahme der Probenflüssigkeit 3 erforderlich ist. Vielmehr kann die geöffnete Plattform 2 mittels des Messgeräts 14 von einem Benutzer sicher gehalten werden.

Nach der Aufnahme von Probenflüssigkeit 3, beispielsweise Blut, erfolgt die Untersuchung bzw. Manipulation der Probenflüssigkeit 3. Beispielsweise strömt die Probenflüssigkeit 3 aufgrund von Kapillarkräften in die Probenkammer 6 und wirkt dort vorzugsweise mit einem voreingebrachten Reagenz derart zusammen, dass insbesondere eine gewünschte Messung bzw. Bestimmung, wie die Glucose-Bestimmung, durch das Messgerät 14, beispielsweise durch eine geeignete optische Methode, erfolgen kann. Hierzu weist das Messgerät 14 eine entsprechende Messeinrichtung 20, wie einen optischen Detektor oder Sensor, und eine Anzeigeeinrichtung 21 zur Darstellung des ermittelten Werts auf.

Anschließend wird die benutzte Plattform (vollständig) vom Träger 10 getrennt und ausgeworfen. Das Messgerät 14 kann hierzu eine entsprechende Trenneinrichtung und Auswurfeinrichtung aufweisen. Beim Darstellungsbeispiel wird dies einfach durch Weiterfördern des Trägers 10 über die in Fig. 4 gezeigte Stellung hinaus erreicht, wie in Fig. 5 angedeutet.

Es ist anzumerken, dass das Separieren bzw. Trennen der benutzten oder zu benutzenden Plattform 2 - vom Träger 10 oder beispielsweise von der nachfolgenden Plattform 2 wahlweise unmittelbar vor, gleichzeitig mit oder nach dem Öffnen der Plattform 2 zur Aufnahme von Probenflüssigkeit 3 und/oder der Aufnahme und/oder Untersuchung der Probenflüssigkeit 3 in der jeweiligen Plattform 2 erfolgen kann.

Das Messgerät 14 ist vorzugsweise mobil, insbesondere tragbar, ausgebildet. Um ein handliches, leichtes Gerät zu ermöglichen, werden die mechanisch auf die Vorrichtung 1 bzw. die einzelnen Plattformen 2 einwirkenden Einrichtungen vorzugsweise nur durch manuelle Betätigung erreicht bzw. angetrieben.

Fig. 6 bis 8 zeigen die Vorrichtung 1 mit einer Vielzahl von Plattformen 2 gemäß einer weiteren Ausführungsform mit einem zugeordneten Messgerät 14.

Die Plattformen 2 hängen hier wieder unmittelbar aneinander, jedoch über verhältnismäßig dünne bzw. stark geschwächte Verbindungen 4. Bedarfsweise können die Plattformen 2 zusätzlich auf einen Träger 10 zur Stabilisierung angeordnet und/oder mit einer durchgehenden bzw. gemeinsamen Abdeckung 8 zur Stabilisierung versehen sein. Jedoch kann die in den Fig. 6 bis 8 nicht dargestellte Abdeckung 8 bedarfsweise auch nicht durchgehend, sondern nur jeweils auf den einzelnen Plattformen 2 vorgesehen sein.

Bei dieser Ausführungsform ist die Probenkammer 6 in einem in Förderrichtung der Plattformen 2 rückwärtigen Teil der jeweiligen Plattform 2 angeordnet. Die Kanäle 5 und 7 enden in einem seitlichen Randbereich der jeweiligen Plattform 2 blind.

Das Messgerät 14 weist hier zwei gegen Federkraft ineinander schiebbare bzw. zueinander verschiebbare Gehäuseteile 22 und 23 auf. Ausgehend von der in Fig. 6 dargestellten Ausgangsposition, ist das Vorderteil 22 nach links - also relativ zur als nächstes zu verwendenden Plattform 2 - verschiebbar. Hierbei ist mittels einer Öffnungseinrichtung 18, die beim Darstellungsbeispiel insbesondere durch eine Schneidkante, Klinge, Schere oder dgl. gebildet ist, betätigbar bzw. bewegbar, um die Plattform 2 derart aufzuschneiden, dass der Aufnahmekanal 5 und der Entlüftungskanal 7 geöffnet werden.

Beim Darstellungsbeispiel erfolgt das Auftrennen bzw. Aufschneiden in Längsrichtung, d.h. in Förderrichtung der Plattform 2 bzw. in Richtung der Längserstreckung der Vorrichtung 1. Hier wird beim Öffnen ein Randstreifen bis zu einer Verjüngung bzw. Hinterschneidung 13 abgetrennt, wobei die die Schnittlinie kreuzenden Kanäle 5 und 7 zwangsläufig geöffnet werden.

Im geöffneten, in Fig. 8 dargestellten Zustand ragt die aufgeschnittene Plattform 2 mit dem Aufnahmekanal 5 derart vor, dass nun Probenflüssigkeit 3 aufnehmbar ist. Die aufgenommene Probenflüssigkeit 3 strömt dann in die beim Darstellungsbeispiel im Bereich des noch in dem Messgerät 14 befindlichen Endes der Plattform 2 gelegenen Probenkammer 6, um die gewünschte Untersuchung bzw. Manipulation mit Messeinrichtung 20 zu ermöglichen. Der ermittelte Wert, kann dann wiederum über die Anzeigeeinrichtung 21 angezeigt werden.

Nach erfolgter Messung wird die benutzte Plattform 2 von den anderen Plattformen 2 separiert. Beim Darstellungsbeispiel wird hierzu das Vorderteil 22 durch Lösen einer nicht dargestellten Sperre und vorzugsweise Federkraft, wieder in die entgegengesetzte Richtung - in Fig. 7 nach rechts - bewegt, wobei die benutzte Plattform 2 durch einen entsprechenden Mitnehmer 24 mit nach vorne, also in Bewegungsrichtung, mitgenommen und dabei von den anderen Plattformen 2 getrennt wird, die durch eine nicht dargestellte Rückhalteeinrichtung zurückgehalten werden. Die benutzte Plattform 2 kann dann entnommen oder ggf. unmittelbar aufgeworfen werden.

Schließlich können die unbenutzten Plattformen 2 durch die Förder- bzw. Vorschubeinrichtung 16, die in den Fig. 6 bis 8 nur sehr schematisch durch eine Feder angedeutet ist, weitergefördert werden, beispielsweise nach Freigabe einer Sperre, so dass die nächste Plattform 2 in die Ausgangsposition gemäß Fig. 6 vorrückt und ggf. sogar das vorgenannte Auswerfen der benutzten Plattform 2 bewirkt.

## Patentansprüche

1. Vorrichtung (1) mit einer Vielzahl von mikrofluidischen Plattformen (2) zur Aufnahme und insbesondere Untersuchung oder Manipulation von Probenflüssigkeit (3), wie Blut zur Glucose-Bestimmung, wobei die Plattformen (2) im verschlossenen Zustand von einer vorzugsweise durchgehenden Abdeckung (8) abgedeckt und auf einem gemeinsamen Träger (10) angeordnet sind, wobei der Träger gleichzeitig als Transportband oder Förderband dient, wobei die Plattformen (2) jeweils einzeln separierbar und einzelweise öffenbar sind und jede Plattform (2) einen Aufnahmekanal (5) zur Aufnahme von Probenflüssigkeit (3) aufweist,
**dadurch gekennzeichnet, dass**
jede Plattform (2) einen Längsrand aufweist, in den sich der Aufnahmekanal (5) und ein Entlüftungskanal (7) erstrecken und in dessen Bereich der Aufnahmekanal (5) und der Entlüftungskanal (7) enden, so dass durch Abtrennen des Längsrandes oder durch Öffnen der Abdeckung (8, 11) im Bereich des Längsrandes der Aufnahmekanal (5) und der Entlüftungskanal (7) geöffnet werden.

2. Vorrichtung nach Anspruch 1, **dadurch gekennzeichnet, dass** jede Plattform (2) separate Öffnungen nach außen zur Aufnahme von Probenflüssigkeit (3) und zur Entlüftung aufweist.

3. Vorrichtung nach einem der vorangehenden Ansprüche, **dadurch gekennzeichnet, dass** die Abdeckung (8) einen separat lösbaren oder öffenbaren Bereich, insbesondere in Form eines Längsstreifens (11) umfasst.

4. Vorrichtung nach einem der vorangehenden Ansprüche, **dadurch gekennzeichnet, dass** die Plattformen (2) über Verbindungen (4) unmittelbar miteinander verbunden sind.

5. Vorrichtung nach einem der vorhergehenden Ansprüche, **dadurch gekennzeichnet, dass** die Abdeckung (8) mindestens eine Kavität, vorzugsweise alle Kavitäten, wie einen Aufnahmekanal (5), eine Probenkammer (6) und/oder einen Entlüftungskanal (7) oder deren Öffnungen der Plattformen (2), abdeckt und/oder dass die Abdeckung (8) aus Folie besteht.

6. Vorrichtung nach einem der vorangehenden Ansprüche, **dadurch gekennzeichnet, dass** die Abdeckung (8) zumindest teilweise, insbesondere einen Streifen (11), öffenbar oder abnehmbar, insbesondere abziehbar ist, so dass die Plattformen (2) bzw. der Kanal (5, 7), die Kanäle (5, 7) oder deren Öffnungen geöffnet wird bzw. werden.

7. Vorrichtung nach einem der vorangehenden Ansprüche, **dadurch gekennzeichnet, dass** die Plattformen (2) bandförmig hintereinander angeordnet sind.

8. Vorrichtung nach einem der vorangehenden Ansprüche 4 bis 7, **dadurch gekennzeichnet, dass** der Träger (10) bandförmig ausgebildet ist.

9. Vorrichtung nach einem der vorangehenden Ansprüche, **dadurch gekennzeichnet, dass** die noch nicht separierten Plattformen (2) aufwickelbar sind, insbesondere aufgrund eigener Flexibilität, aufgrund einer flexiblen gegenseitigen Verbindung (4) und/oder aufgrund einer Flexibilität des Trägers (10) und/oder dass die noch nicht separierten Plattformen (2) stapelbar sind, insbesondere aufgrund einer flexiblen gegenseitigen Verbindung (4) und/oder aufgrund einer Flexibilität des Trägers (10).

10. Vorrichtung nach einem der vorangehenden Ansprüche, **dadurch gekennzeichnet, dass** die Vorrichtung (1), insbesondere die Plattformen (2), mit mechanischen und/oder optischen Markierungen, Aussparungen, Löchern, Verjüngungen, Schwächungen oder Hinterschneidungen (12) versehen sind, um das Fördern, Positionieren, Erfassen und/oder Separieren einzelner Plattformen (2) zu ermöglichen.

11. Messgerät (14) zur Untersuchung von Probenflüssigkeit (3), wie Blut zur Glucose-Bestimmung mittels einer mikrofluidischen Plattform (2), **dadurch gekennzeichnet, dass** das Messgerät (14) eine Vorrichtung gemäß einem der voranstehenden Ansprüche enthält.

12. Messgerät nach Anspruch 11, **dadurch gekennzeichnet, dass** das Messgerät (14) ein vorzugsweise wechselbares Magazin (15) zur Aufnahme der Vorrichtung (1) bzw. Plattformen (2), eine Vorschubeinrichtung (16) zur Förderung der Plattformen (2), eine Öffnungseinrichtung (18) zum einzelweisen Öffnen der Plattformen (2) zur Aufnahme von Probenflüssigkeit (3), eine Trennvorrichtung zur einzelweisen Separation der Plattformen (2) und/oder eine Auswurfeinrichtung zur einzelweisen Abgabe einer benutzten Plattform (2) aufweist.

13. Verfahren zur Untersuchung oder Manipulation von Probenflüssigkeit (3), wie Blut zur Glucose-Messung, mittels einer mikrofluidischen, die Probenflüssigkeit (3) aufnehmenden Plattform (2), insbesondere mittels einer Vorrichtung gemäß einem der vorhergehenden Ansprüche, wobei eine Vielzahl von mittels einer Abdeckung (8) abgeschlossenen Plattformen (2) mittels eines gemeinsamen Trägers (10) gefördert werden, wobei die Plattformen (2) einzelweise und nur bedarfsweise zur unmittelbar anschließenden Untersuchung oder Manipulation von Probenflüssigkeit (3) geöffnet und separiert werden, **dadurch gekennzeichnet, dass** Aufnahmekanäle (5) und Entlüfungskanäle (7) in den Plattformen (2) einzelweise durch Abtrennen eines Längsrandes der Plattformen (2) oder durch Öffnen der Abdeckung (8) im Bereich des Längsrandes der Plattform (2) geöffnet werden.

14. Verfahren nach Anspruch 13, **dadurch gekennzeichnet, dass** die Plattformen (2) einzelweise durch zumindest teilweises Abheben oder Abziehen der Abdeckung (8) geöffnet werden.

15. Verfahren nach Anspruch 13, **dadurch gekennzeichnet, dass** ein Randstreifen bis zu einer Verjüngung bzw. Hinterscheidung (13) abgetrennt wird, so dass die die Schnittlinie kreuzende Kanäle zwangsläufig geöffnet werden.

16. Verfahren nach einem der vorhergehenden Ansprüche 13 bis 15, **dadurch gekennzeichnet, dass** nach einer Förderbewegung die Plattform (2) aus dem Messgerät (14) derart hervorragt, dass nun Probenflüssigkeit (3) durch einen Aufnahmekanal (5) aufnehmbar ist.

## Claims

1. Apparatus (1) having a plurality of microfluidic platforms (2) for receiving and in particular investigating or manipulating sample liquid (3), such as blood for measuring glucose levels, wherein the platforms (2) in the closed state are covered by a preferably continuous cover (8) and arranged on a common carrier (10), the carrier simultaneously serving as a transporter or conveyor belt, wherein the platforms (2) can each be individually detached and individually opened and each platform (2) has a receiving channel (5) for receiving sample liquid (3),
**characterised in that**
each platform (2) has a longitudinal edge into which the receiving channel (5) and a venting channel (7) extend and in the region of which the receiving channel (5) and the venting channel (7) end, so that by severing the longitudinal edge or by opening the cover (8, 11) in the region of the longitudinal edge, the receiving channel (5) and the venting channel (7) are opened.

2. Apparatus according to claim 1, **characterised in that** each platform (2) comprises separate openings to the outside for receiving sample liquid (3) and for venting.

3. Apparatus according to one of the preceding claims, **characterised in that** the cover (8) comprises a separately detachable or openable region, particularly in the form of a longitudinal strip (11).

4. Apparatus according to one of the preceding claims, **characterised in that** the platforms (2) are directly connected to one another by means of connections (4).

5. Apparatus according to one of the preceding claims, **characterised in that** the cover (8) covers at least one cavity, preferably all the cavities, such as a receiving channel (5), a sample chamber (6) and/or a venting channel (7) or the openings thereof in the platforms (2), and/or the cover (8) consists of film.

6. Apparatus according to one of the preceding claims, **characterised in that** the cover (8) is at least partially adapted to be opened or removed, particularly pulled off, particularly a strip (11), so that the platforms (2) or the channel (5, 7), the channels (5, 7) or the openings thereof is or are opened.

7. Apparatus according to one of the preceding claims, **characterised in that** the platforms (2) are arranged in a strip one behind the other.

8. Apparatus according to one of the preceding claims 4 to 7, **characterised in that** the carrier (10) is configured as a strip.

9. Apparatus according to one of the preceding claims, **characterised in that** the platforms (2) that are not yet detached can be wound in a coil, particularly by virtue of their inherent flexibility, a flexible mutual connection (4) and/or a flexibility of the carrier (10), and/or the platforms (2) that are not yet detached can be stacked, particularly by virtue of a flexible mutual connection (4) and/or a flexibility of the carrier (10).

10. Apparatus according to one of the preceding claims, **characterised in that** the apparatus (1), particularly the platforms (2), are provided with mechanical and/or optical markings, cut-outs, holes, tapers, weakened points or undercuts (12), to allow the conveying, positioning, gripping and/or detachment of individual platforms (2).

11. Measuring equipment (14) for examining sample liquid (3), such as blood for measuring glucose levels, by means of a microfluidic platform (2), **characterised in that** the measuring equipment (14) contains an apparatus according to one of the preceding claims.

12. Measuring equipment according to claim 11, **characterised in that** the measuring equipment (14) comprises a preferably replaceable magazine (15) for receiving the apparatus (1) or platforms (2), an advancing device (16) for conveying the platforms (2), an opening device (18) for individually opening the platforms (2) to receive sample liquid (3), a severing device for individually separating the platforms (2) and/or a disposal device for individually discarding a used platform (2).

13. Process for investigating or manipulating sample liquid (3) such as blood for measuring glucose levels, by means of a microfluidic platform (2) that receives the sample liquid (3), in particular by means of an apparatus according to one of the preceding claims, wherein a plurality of platforms (2) closed off by a cover (8) are delivered by means of a common carrier (10), wherein the platforms (2) are opened and detached individually and only as required for the immediate investigation or manipulation of sample liquid (3), **characterised in that** receiving channels (5) and venting channels (7) in the platforms (2) are individually opened by severing a longitudinal edge of the platforms (2) or by opening the cover (8) in the region of the longitudinal edge of the platform (2).

14. Process according to claim 13, **characterised in that** the platforms (2) are opened individually by at least partial lifting or pulling off of the cover (8).

15. Process according to claim 13, **characterised in that** an edge strip is severed up to a tapering or undercut (13), so that the channels intersecting with the cutting line are automatically opened.

16. Process according to one of the preceding claims 13 to 15, **characterised in that** after a conveying movement the platform (2) projects from the measuring equipment (14) such that sample liquid (3) can now be received through a receiving channel (5).

## Revendications

1. Dispositif (1) présentant une multiplicité de plateformes microfluidiques (2) pour la réception et en particulier l'analyse ou la manipulation d'un liquide de prélèvement (3) tel que le sang pour la détermination du glucose, les plateformes (2) étant recouvertes à l'état fermé par un couvercle de préférence continu (8) et étant disposées sur un support commun (10), le support servant en même temps de bande de transport ou de tapis roulant, les plateformes (2) pouvant respectivement être séparables individuellement et pouvant être ouvertes individuellement et chaque plateforme (2) présentant un canal de réception (5) pour recevoir un fluide de prélèvement (3), **caractérisé en ce que** chaque plateforme (2) présente un bord longitudinal dans lequel s'étendent le canal de réception (5) et un canal d'aération (7) et dans la zone duquel se terminent le canal de réception (5) et le canal d'aération (7) de sorte que par séparation du bord longitudinal ou par ouverture du couvercle (8, 11) dans la zone du bord longitudinal, le canal de réception (5) et le canal d'aération (7) soient ouverts.

2. Dispositif selon la revendication 1, **caractérisé en ce que** chaque plateforme (2) présente des ouvertures séparées vers l'extérieur pour la réception de fluide de prélèvement (3) et pour l'aération.

3. Dispositif selon l'une des revendications précédentes, **caractérisé en ce que** le couvercle (8) comprend une zone pouvant être séparément détachée ou ouverte, en particulier sous la forme d'une bande longitudinale (11).

4. Dispositif selon l'une des revendications précédentes, **caractérisé en ce que** les plateformes (2) sont reliées les unes aux autres par des liaisons (4).

5. Dispositif selon l'une des revendications précédentes, **caractérisé en ce que** le couvercle (8) recouvre au moins une cavité, de préférence, toutes les cavités telles qu'un canal de réception (5), une chambre d'échantillon (6) et/ou un canal d'aération (7) ou leurs ouvertures des plateformes (2) et/ou **en ce que** le couvercle (8) consiste en une feuille.

6. Dispositif selon l'une des revendications précédentes, **caractérisé en ce que** le couvercle (8), en particulier d'une bande (11) peut être ouvert ou ôté au moins partiellement, en particulier, peut être retiré, de sorte que les plateformes (2) ou le canal (5, 7), les canaux (5, 7) ou leurs ouvertures puissent être ouverts.

7. Dispositif selon l'une des revendications précédentes, **caractérisé en ce que** les plateformes (2) sont disposées en bande les unes derrière les autres.

8. Dispositif selon l'une des revendications 4 à 7, **caractérisé en ce que** le support (10) est en forme de bande.

9. Dispositif selon l'une des revendications précédentes, **caractérisé en ce que** les plateformes (2) non encore séparées peuvent être enroulées, en particulier en raison de leur propre flexibilité, en raison d'une liaison réciproque flexible (4) et/ou en raison d'une flexibilité du support (10) et/ou **en ce que** les plateformes non encore séparées (2) peuvent être empilées, en particulier en raison d'une liaison réciproque flexible (4) et/ou en raison d'une flexibilité du support (10).

10. Dispositif selon l'une des revendications précédentes, **caractérisé en ce que** le dispositif (1), en particulier les plateformes (2) sont dotées de marquages mécaniques et/ou optiques, d'évidements, de trous, des rétrécissements, de zones fragiles ou de contre-découpes (12) pour permettre l'acheminement, le positionnement, la saisie et/ou la séparation de plateformes individuelles (2).

11. Appareil de mesure (14) pour l'analyse de fluide de prélèvement (3) tel que le sang pour la détermination du glucose au moyen d'une plateforme microfluidique (2), **caractérisé en ce que** l'appareil de mesure (14) contient un dispositif selon l'une des revendications précédentes.

12. Appareil de mesure selon la revendication 11, **caractérisé en ce que** l'appareil de mesure (14) présente un chargeur échangeable (15) pour la réception du dispositif (1) ou des plateformes (2), un agencement d'avancée (16) pour l'acheminement des plateformes (2), un agencement d'ouverture (18) pour l'ouverture individuelle des plateformes (2) pour la réception du fluide de prélèvement (3), un dispositif de séparation pour la séparation individuelle des plateformes (2) et/ou un agencement d'éjection pour le retrait individuel d'une plateforme utilisée (2).

13. Procédé d'analyse ou de manipulation de fluide de prélèvement (3) tel que le sang pour la mesure du glucose, au moyen d'une plateforme (2) de réception de fluide de prélèvement (3) microfluidique, en particulier au moyen d'un dispositif selon l'une des revendications précédentes, dans lequel une multiplicité de plateformes (2) fermées par un couvercle (8) est acheminée au moyen d'un support commun (10), les plateformes (2) étant ouvertes et séparées individuellement et uniquement si besoin pour l'analyse ou la manipulation immédiatement consécutive de fluide de prélèvement (3), **caractérisé en ce que** les canaux de réception (5) et les canaux d'aération (7) dans les plateformes (2) sont ouverts individuellement par séparation d'un bord longitudinal des plateformes (2) ou par ouverture du couvercle (8) dans la zone du bord longitudinal de la plateforme (2).

14. Procédé selon la revendication 13, **caractérisé en ce que** les plateformes (2) sont ouvertes individuellement par soulèvement ou retrait au moins partiel du couvercle (8).

15. Procédé selon la revendication 13, **caractérisé en ce qu'**une bande périphérique est séparée jusqu'à un rétrécissement ou une contre-découpe (13), de sorte que les canaux croisant la ligne de section soient ouverts par la force des choses.

16. Procédé selon l'une des revendications 13 à 15 précédentes, **caractérisé en ce qu'**après un mouvement d'avancée, la plateforme (2) dépasse de l'appareil de mesure (14) de telle sorte qu'un fluide de prélèvement (3) puisse alors être reçu par un canal de réception (5).
